# EUROPÄISCHE PATENTANMELDUNG

(11) **EP 4 272 872 A1**
(43) Veröffentlichungstag der Anmeldung: **08.11.2023**
(21) Anmeldenummer: 22171432.2
(22) Anmeldetag: 03.05.2022
(51) Int. Cl.: B05B 1/30

(54) **AUSTRAGKOPF FÜR PHARMAZEUTISCHE ODER KOSMETISCHE FLÜSSIGKEITEN**

(71) Anmelder: Aptar Radolfzell GmbH, 78315 Radolfzell (DE)
(72) Erfinder: Greiner-Perth, Jürgen, 78244 Gottmadingen (DE); Ritsche, Stefan, 78253 Eigeltingen (DE)
(74) Vertreter: Witte, Weller und Partner Patentanwälte mbB Stuttgart

(57) **Zusammenfassung**

Vorgeschlagen wird ein Austragkopf (20) für pharmazeutische oder kosmetische Flüssigkeiten mit einer Basiseinheit (40) zur Anbringung an einem Flüssigkeitsspeicher (12) und mit einer Austrageinheit (60), die über ein Applikatorgehäuse (64) verfügt, welches von einer Austragöffnung (62) durchdrungen ist. Die Austrageinheit (60) ist gegen die Kraft einer Rückstellfeder (80) gegenüber der Basiseinheit (40) verlagerbar, wobei eine Pumpeinrichtung (50) des Austragkopfes (20) durch die Bewegung der Austrageinheit (60) gegenüber der Basiseinheit (40) betätigbar ist und in Reaktion auf die Betätigung Flüssigkeit aus dem Flüssigkeitsspeicher (12) zur Austragöffnung (62) fördert. Die Rückstellfeder (80) ist als Kunststofffeder ausgebildet, vorzugsweise als Kunststoffbalgfeder.

## Beschreibung

### ANWENDUNGSGEBIET UND STAND DER TECHNIK

Die Erfindung betrifft einen Flüssigkeitsspender und einen hierfür vorgesehenen Austragkopf für pharmazeutische oder kosmetische Flüssigkeiten. Der Austragkopf verfügt über eine Basiseinheit zur Anbringung an einem Flüssigkeitsspeicher sowie über eine gegenüber der Basiseinheit verlagerbaren Austrageinheit. Die Austrageinheit weist eine Austragöffnung auf, durch die hindurch Flüssigkeit abgegeben werden kann. Der Austragkopf weist eine Pumpeinrichtung auf, die durch Niederdrücken der Austrageinheit in Richtung der Basiseinheit betätigt werden kann und mittels derer Flüssigkeit ausgetragen werden kann, die zuvor aus dem Flüssigkeitsspender in eine Pumpkammer der Pumpeinrichtung gesogen wurden.

Nach dem Niederdrücken der Austrageinheit zum Zwecke des Austrags der Flüssigkeit kehrt die Austrageinheit im Zuge einer Rückstellbewegung in ihre Ausgangsstellung zurück, in der sie von der Basiseinheit beabstandet ist. Hierbei wird Flüssigkeit aus dem Flüssigkeitsspeicher in die Pumpkammer eingesogen, die beim nächsten Niederdrücken der Austrageinheit zumindest teilweise ausgetragen wird. Der Austragkopf weist eine Rückstellfeder auf, die beim Niederdrücken der Austrageinheit gespannt wird und anschließend die Rückstellbewegung bewirkt.

Gattungsgemäße Flüssigkeitsspender sind üblicherweise als Einwegspender ausgebildet. Nach Verbrauch der Flüssigkeit im Flüssigkeitsspeicher werden sie entsorgt.

### AUFGABE UND LÖSUNG

Aufgabe der Erfindung ist es, einen Flüssigkeitsspender der oben beschriebenen Art oder dessen Austragkopf derart zu gestalten, dass ein hohes Maß an Recyclingfähigkeit erzielt wird.

Vorgeschlagen wird hierzu erfindungsgemäß ein Austragkopf für pharmazeutische oder kosmetische Flüssigkeiten sowie ein unbefüllter oder befüllter Flüssigkeitsspender, der einen Flüssigkeitsspeicher sowie einen Austragkopf der genannten Art umfasst.

Der Austragkopf erfindungsgemäßer Art umfasst in der oben beschriebenen Weise eine Basiseinheit, die mit dem Flüssigkeitsspeicher verbunden ist, insbesondere mittels einer Schraub- oderSchnappverbindung. Auch eine einstückige Verbindung von Basiseinheit und Flüssigkeitsspeicher ist möglich.

Gegenüber dieser Basiseinheit ist die Austrageinheit des Austragkopfes verlagerbar, vorzugsweise linear verlagerbar, insbesondere in einer mit einer Mittelachse der Basiseinheit und/oder des Flüssigkeitsspeichers übereinstimmenden Richtung. Die Kraftbeaufschlagung der Austrageinheit gegenüber der Basiseinheiterfolgt manuell. Vorzugsweise umgreift der Benutzer die Basiseinheit oder den Flüssigkeitsspeicher und drückt die Austrageinheit mittels Zeigefinger und/oder Mittelfinger herunter.

Zwischen der Basiseinheit und der Austrageinheit ist die Pumpeinrichtung derart angeordnet, dass sie durch die Relativverlagerung von Basiseinheit und Austrageinheit betätigt wird. Die Pumpeinrichtung weist vorzugsweise eine Pumpkammer auf. Ein mit einem Einlassventil versehener Einlasskanal verbindet die Pumpkammer mit dem Flüssigkeitsspeicher. Ein mit einem Auslassventil versehener Auslasskanal verbindet die Pumpkammer mit der Austragöffnung, die ein Applikatorgehäuse der Austrageinheit durchdringt. Beim Niederdrücken der Austrageinheit wird das Einlassventil geschlossen und das Auslassventil geöffnet, so dass die Flüssigkeit austritt. Beim darauffolgenden Rückhub schließt das Auslassventil und das Einlassventil öffnet, so dass die sich hierbei vergrößernde Pumpkammer Flüssigkeit aus dem Flüssigkeitsspeicher ansaugt, vorzugsweise mittels eines Steigrohres, welches in den Flüssigkeitsspeicher ragt, oder aus einem kollabierenden Beutel innerhalb des Flüssigkeitsspeichers.

Der erfindungsgemäße Austragkopf ist vorzugsweise einfach recyclebar. Insbesondere kann er vollständig oder annähernd vollständig (>99%) aus Kunststoff bestehen, vorzugsweise aus Kunststoffen, die in einem gemeinsamen Recyclingprozess verarbeitet werden können. Auch die Rückstellfeder, die zwischen der Basiseinheit und der Austrageinheit wirkt, ist erfindungsgemäß als Kunststofffeder ausgestaltet.

Insbesondere kann es sich bei der Rückstellfeder um eine Balgfeder handeln, also einen umlaufend geschlossenen Faltenbalg, der mit seinen beiden Enden an Flächen der Austrageinheit und der Basiseinheit anliegt. Beim Niederdrücken der Austrageinheit wird die Balgfeder axial zusammengedrückt und dabei elastisch gespannt. Entfällt die niederdrückende Kraft, so entspannt die Balgfeder und drückt die Austrageinheit wieder von der Basiseinheit weg. Ein bevorzugtes Material für die Balgfeder ist TPE (thermoplastisches Elastomer).

Grundsätzlich ist es möglich, die Rückstellfeder aus Kunststoff innerhalb eines von der Austrageinheit und der Basiseinheit umgebenen Innenraum anzuordnen, so dass sie von außen nicht sichtbar ist. Wird dieser Weg gewählt, so können Austragköpfe, die ursprünglich für die Aufnahme von metallischen Schraubenfedern vorgesehen waren, stattdessen mit Kunststoff-Balgfedern ausgerüstet werden, die in denselben Aufnahmeraum eingesetzt werden, in dem bei anderen Varianten metallische Schraubenfedern eingesetzt sind. Dies gestattet es, viele oder sogar alle anderen Teile zwischen zwei Varianten des Austragkopfes mit Metallfeder und Kunststofffeder identisch zu gestalten.

Vorzugsweise ist jedoch vorgesehen, dass die Rückstellfeder an einer Außenseite der Austrageinheit angebracht ist und im regulären Betrieb daherzu sehen ist. Hierdurch wird der Bedarf vermieden, den durch die Basiseinheit und die Austrageinheit definierten Innenraum ausreichend groß für eine Balgfeder im entspannten und im komprimierten Zustand bereitzuhalten.

Insbesondere ist die Rückstellfeder aus Kunststoff so angeordnet, dass sie zumindest abschnittsweise Außenflächen der Basiseinheit und der Austrageinheit umhüllt. Es wird auch in einem solchen Falle als vorteilhaft angesehen, wenn die Austrageinheit und die Basiseinheit in einem von diesen Elementen gemeinsam umschlossenen Innenraum einen Federaufnahmeraum aufweisen, der zur Aufnahme einer Schraubenfeder ausgebildet ist, so dass statt der Rückstellfeder aus Kunststoff auch eine Schraubenfeder aus Metall verwendet werden kann.

Allerdings ist bei der Gestaltung mit Kunststofffedern im Federaufnahmeraum vorzugsweise keine Schraubenfeder angeordnet und der Federaufnahmeraum verbleibt leer. Er dient somit nur der Aufnahme einer metallischen Schraubenfeder, wenn die weitgehend baugleiche Austrageinheit und die weitgehend baugleiche Basiseinheit bei einer anderen Variante ohne Kunststofffeder ausgebildet sind. Vorzugsweise sind an der Basiseinheit und der Austrageinheit jeweils ringförmige Anlageflächen für die Schraubenfeder vorgesehen, wobei die ringförmigen Anlageflächen vorzugsweise innenseitig und/oder außenseitig durch Führungsflächen flankiert sind.

Denkbar ist auch eine Variante, bei der sowohl eine innenliegende metallische Schraubenfeder als auch eine außenliegende Kunststofffeder, insbesondere eine Balgfeder, vorgesehen sind, so dass eine erhöhte gemeinsame Federkraft erzielt wird.

Ist die Kunststofffeder an der Außenseite des Austragkopfes vorgesehen, so stellt vorzugsweise eine Oberseite der Basiseinheit ein Krafteinkopplungsfläche bereit.

Die gegenüberliegende Krafteinkopplungsfläche auf der Seite der Austrageinheit wird vorzugsweise durch eine Fingerauflage gebildet. Diese Fingerauflage ist an der Austrageinheit zum Zwecke der manuellen Kraftbeaufschlagung der Austrageinheit in Richtung der Basiseinheit vorgesehen. Insbesondere vorzugsweise handelt es sich um eine Fingerauflage mit ringförmiger Form, die das Applikatorgehäuse der Austrageinheit umgibt. Die Fingerauflage kann insbesondere von umlaufend gleichbleibender Breite sein oder über zwei gegenüberliegende Betätigungsflügel zur Auflage von Zeigefinger und Mittelfinger verfügen.

Die Rückstellfederwirktvorzugsweise auf diese Fingerauflage und liegt hierzu insbesondere an einer Unterseite der Fingerauflage an oder ist hier befestigt.

Die Fingerauflage kann einstückig am Applikatorgehäuse angeformt sein. Demgegenüber bevorzugt ist es allerdings, wenn es sich um ein separates Bauteil handelt, welches am Applikatorgehäuse befestigt wird, insbesondere mittels einer Schnappverbindung. Dies gestattet es, unterschiedliche Fingerauflagen für Versionen des Austragkopfes mit und ohne außenliegende Kunststofffeder zu verwenden.

Die Fingerauflage kann weiterhin einstückig mit der Kunststofffeder ausgebildet sein. Eine mögliche Bauweise sieht dabei vor, dass der die Fingerauflage bildende Teil und der die Kunststofffeder bildende Teil aus einheitlichem identischem Material bestehen, beispielsweise einem TPE-Kunststoff. Das Material ist dabei primär anhand der gewünschten Verformungscharakteristik der Kunststofffeder ausgelegt. Der die Fingerauflage bildende Teil ist vorzugsweise durch eine größere Wandstärke als der Federteil und/oder durch zusätzliche Verrippung so ausgesteift, dass er nur in akzeptablem Maße zur Verformung neigt.

Eine einstückige Gestalt der Fingerauflage mit der Kunststofffeder kann aber auch durch eine stoffschlüssige Verbindung zweier unterschiedlicher Kunststoffmaterialien gebildet sein. Ein starrerer Teil bildet dabei zumindest einen Teil der Oberseite der Fingerauflage, während ein elastischerer Teil den Federteil des einstückigen Bauteils bildet. Der starre Teil kann zusätzlich einstückig mit dem Applikatorgehäuse ausgebildet sein, kann aber auch als getrenntes Teil ausgebildet sein. Die Herstellung einer stoffschlüssigen Verbindung erfolgt vorzugsweise durch Mehrkomponentenspritzguss, wobei in eine erweiterbare Spritzgusskavität nacheinander zunächst ein erster Kunststoff und dann nach Vergrößerung der Kavität ein zweiter Kunststoff eingebracht wird. In einer Grenzzone gehen die Kunststoffe eine innige und luftdichte Verbindung ein. Bei einer einstückigen Gestaltung aus mehreren Kunststoffen kann es von Vorteil sein, wenn die Grenzfläche so geformt ist, dass die Teile aus verschiedenen Kunststoffen zusätzlich zum Stoffschluss auch mittels Hinterschneidungen formschlüssig miteinander verbunden sind.

Die einstückige Gestaltung der Fingerauflage mit der Kunststofffeder vereinfacht die Montage, da durch Anbringung der Fingerauflage am Applikatorgehäuse auch gleichzeitig die Feder in ihre Sollposition gebracht wird. Gleiches ist aber auch mit einer zweistückigen Gestaltung zu erreichen, bei der die Fingerauflage und die Kunststofffeder getrennt hergestellte Teile sind, die miteinander vor Anbringung am Applikatorgehäuse bereits kraft- oder formschlüssig verbunden werden, beispielsweise durch eine Klemmverbindung.

Sofern die Fingerauflage und die Rückstellfeder als getrennte Bauteile ausgebildet sind, ist es von Vorteil, wenn an der Fingerauflage und insbesondere an deren Unterseite eine Zentrierungsgeometrie vorgesehen ist, die eine Krafteinkopplungsfläche der Rückstellfeder von innen und/oder außen führt und vorzugsweise auch der kraft- oder formschlüssigen Kopplung von Rückstellfeder und Fingerauflage dient. Sind die Fingerauflage und die Rückstellfeder als separate Teile ausgebildet und ist die Rückstellfeder als Balgfeder ausgestaltet, so ist es von Vorteil, wenn die an der Fingerauflage anliegende Seite der Balgfeder einen nach außen oder vorzugsweise nach innen wirkenden Kragen aufweist, der die Balgsegmente durchmesserbezogen um mindestens 5%, vorzugsweise um mindestens 10%, nach außen bzw. innen überragt. Dieser Kragen erleichtert bei der Herstellung der Balgfeder die Entformung.

Vorzugsweise die genannte Fingerauflage, je nach Bauweise jedoch auch ein anderes Teil der Austrageinheit oder der Basiseinheit, kann Träger der Rückstellfeder sein. Dies bedeutet, dass die Rückstellfeder mit diesem als Träger agierenden Bauteil fest verbunden ist. Hier kommen sowohl Klemmverbindungen als auch einstückige Verbindungen in Frage, insbesondere stoffschlüssige Verbindungen, bei denen das als Träger agierende Bauteil und die Rückstellfeder aus unterschiedlichen Materialen bestehen, jedoch durch Mehrkomponentenspritzguss unlösbar und innig miteinander verbunden sind.

Wird der Träger montiert, so wird hierdurch auch die Rückstellfeder in ihre Sollposition gebracht. Dies erleichtert die Montage der Rückstellfeder. An anderen Bauteilen als am Trägerbauteil ist die Rückstellfeder nicht fest montiert, sondern kommt mit diesen lediglich während der Montage oder der Betätigung in Kontakt.

Die Verwendung einer Kunststofffeder, insbesondere einer Balgfeder, führt verglichen mit einer metallischen Rückstellfeder zu einer tendenziell geringeren Rückstellkraft. Die Austrageinheit gelangt daher nicht mit gleicher Zuverlässigkeit in die unbetätigte Ausgangslage wie im Falle einer metallischen Rückstellfeder. Um trotz des sich dadurch ergebenden potentiell verringerten Hubweges eine reproduzierbare Austragmenge zu gewährleisten, kann vorgesehen sein, dass die Pumpeinrichtung derart ausgebildet und/oder derart an die Austrageinheit gekoppelt ist, dass ausgehend von der Ausgangslage eine Verlagerung der Austrageinheit in Richtung der betätigten Endlage für einen Teilhubweg kein Flüssigkeitsaustrag bewirkt wird. Ausgehend von der Endlage führt die beginnende Betätigung daher zunächst nicht zu einem Austrag, vorzugsweise indem zunächst noch ein Rückfluss von Flüssigkeit aus der Pumpkammer in den Flüssigkeitsspeicher möglich ist. Während der Betätigung wird der Rückfluss erst nach einem kurzen Teilhubweg verschlossen, vorzugsweise nach mindestens 3% des Gesamthubweges, insbesondere nach mindestens 6% des Gesamthubweges. Erst nach Zurücklegen dieses Teilhubweges ist die Pumpkammer isoliert und der Austrag beginnt.

Somit reicht ein unvollständiger Rückhub, um dennoch bei der nächsten Pumpenbetätigung die durch die Pumpeinrichtung baulich definierte Flüssigkeitsmenge reproduzierbar auszugeben.

Bevorzugt weist der Austragkopf ein Einlassventil auf, das als Schiebeventil ausgebildet ist. Dieses Schiebeventil verfügt über einen vorzugsweise ortsfest an der Basiseinheit vorgesehenen Ventilkanal sowie über einen vorzugsweise ortsfest an der Austrageinheit vorgesehenen Ventilschieber, der beim Austrag bestimmungsgemäß in den Ventilkanal einfährt und der zur umfänglichen Abdichtung mit einer Innenseite des Ventilkanals ausgebildet ist. Durch ein solches Ventil lassen sich besonders gut der oben beschriebene verzögerte Austrag und die stets einheitliche Dosiermenge realisieren. Die Pumpkammer wird bei Betätigung in jener Zwischenstellung gegenüber dem Flüssigkeitsspeicher isoliert, in der es zur umfänglichen Anlage des Ventilschiebers im Ventilkanal kommt.

Das Auslassventil des Austragkopfes öffnet vorzugsweise druckgesteuert, so dass es bei Überdruck in der Pumpkammer öffnet. Das Auslassventil ist vorzugsweise als "Tip Seal"-Ventil ausgestaltet, das unmittelbar an der Austragöffnung abdichtet, so dass es das Eindringen von Keimen in den Austragkopf wirksam verhindert. Dieses "Tip Seal"-Ventil ist vorzugsweise das einzige Auslassventil. Es sind jedoch auch Gestaltungen umfasst, bei denen der Austragkopf ein erstes Auslassventil aufweist, welches sich unmittelbar an die Pumpkammer anschließt, und ein zweites Auslassventil stromabwärts des ersten Auslassventils aufweist, welches in beschriebener Weise als "Tip Seal"-Ventil ausgebildet und unmittelbar an der Austragöffnung angeordnet ist.

Das vorzugsweise nur eine Auslassventil verfügt als druckabhängig öffnendes Ventil über einen Ventilkörper, der von einer Ventilfeder in eine Schließposition gedrückt wird. Diese Ventilfeder ist vorzugsweise als Kunststofffeder ausgebildet, um das einheitliche Recycling des Austragkopfes zu ermöglichen. Die Ventilfeder kann ebenfalls als Balgfeder ausgebildet sein. Da die Ventilfeder des Auslassventils jedoch keinen großen Federweg benötigt, werden andere Gestaltungen als vorteilhaft angesehen, insbesondere eine Gestaltung, bei der die Ventilfeder durch einen Federrohrkörper gebildet wird, der mittels den Rohrkörper durchdringender Durchbrechungen eine elastische axiale Verkürzung gestattet. Die Durchbrechungen sind vorzugsweise in Form von quer zur Komprimierungsrichtung eingebrachten Schlitzen ausgebildet.

Vorzugsweise ist das Auslassventil derart ausgebildet, dass zu Beginn einer Öffnungsbewegung zum Zwecke des Öffnens ein Druckmaximum benötigt wird, wobei nach dem Öffnen das Auslassventil mit gegenüber dem Druckmaximum verminderten Flüssigkeitsdruck offen gehalten werden kann. Das Auslassventil ist daher vorzugsweise baulich so gestaltet, dass es bei ansteigendem Druck zunächst nicht öffnet. In dieser Phase reicht ein Druckniveau zum Öffnen des Ventils nicht aus, das nach Öffnen in der Lage wäre, das Ventil offenzuhalten. Erreicht wird dieses Verhalten vorzugsweise dadurch, dass der Ventilkörper und eine Anlagefläche der Austrageinheit im geschlossenen Zustand des Auslassventils aneinander anliegen und durch zwischen ihnen herrschende Haftreibung dem Öffnen entgegenwirken. Erst wenn ein ausreichend hoher Druck erzielt wird, wird die Haftreibung überwunden und das Auslassventil öffnet schlagartig. Diese Öffnungscharakteristik verhindert, dass das Ventil beim Öffnen zunächst nur einen engen Auslassschlitz öffnet, der die Austragscharakteristik verschlechtert.

Die Ventilfeder und der Ventilkörper des Auslassventils sind vorzugsweise durch ein einstückiges Kunststoffteil gebildet, um die Montage des Austragkopfes zu erreichen. Insbesondere bestehen sie vorzugsweise aus einem Polyolefin-Kunststoff, insbesondere aus Polyethylen.

Damit sich mit fortschreitendem Austrag von Flüssigkeit aus dem Flüssigkeitsspeicher kein Unterdruck im Flüssigkeitsspeicher aufbaut, stellt der Austragkopf vorzugsweise einen Belüftungspfad zur Verfügung, entlang dessen Ausgleichsluft aus einer umgebenden Atmosphäre in den Flüssigkeitsspeicher strömen kann. Der Belüftungspfad dann dabei insbesondere durch eine Belüftungskammer geführt sein, die außenseitig durch die Rückstellfeder in Form einer Balgfeder umgeben ist.

Damit Luft von außen hier einströmen kann, ist in der Balgfeder vorzugsweise eine Belüftungsöffnung vorgesehen, die einen lichten Querschnitt von mindestens 1 mm² aufweist, insbesondere vorzugsweise von mindestens 5 mm². Es kann sich insbesondere um eine endseitige Aussparung des Balgkörpers handeln. Es kann aber auch sinnvoll sein, das Eindringen von Luft in die Belüftungskammer zu erschweren oder im Falle eines anderen Verlaufs des Belüftungspfades vollständig zu unterbinden, damit die Rückstellfeder einen Luftfederraum umgibt, innerhalb dessen Luft enthalten ist, die bei Verlagerung der Austrageinheit an die Basiseinheit komprimiert wird. Diese Luft wirkt dann als zusätzliche Rückstellfeder.

Um die Neigung der Flüssigkeit im Flüssigkeitsspeicher zu verringern, durch den Belüftungspfad nach außen zu gelangen, kann der Belüftungspfad zumindest abschnittsweise durch einen Kapillarkanal gebildet sein, der stromaufwärts oder stromabwärts der Belüftungskammer vorgesehen sein kann. Ist der Kapillarkanal bezogen auf eine Einströmrichtung stromaufwärts der Belüftungskammer vorgesehen, so wird der Kapillarkanal vorzugsweise zumindest teilweise durch die Balgfeder begrenzt.

Der genannte Belüftungskanal kann zusätzlich eine Filtereinheit aufweisen, die vorzugsweise bezogen auf die Einströmrichtung der Luft stromabwärts der Belüftungskammer vorgesehen ist und die das Eindringen von Keimen verhindert oder vermindert.

Der Austragkopf ist bei einer bevorzugten Gestaltungals Austragkopfzur nasalen Applikation vorgesehen. Das Applikatorgehäuse weist hierfür zumindest in einem distalen Bereich eine längliche und sich zu einem distalen Ende hin verjüngende Formgebung auf. Zur Betätigung ist vorzugsweise eine Fingerauflage seitlich am Applikatorgehäuse vorgesehen, insbesondere eine umlaufende Fingerauflage oder eine Fingerauflage mit zwei Flügeln zur Auflage des Zeigefingers und des Mittelfingers, wie sie oben schon beschrieben wurde. Eine Austragrichtung der Austragöffnung ist bei einem solchen Nasalapplikator vorzugsweise parallel zur Verlagerungsrichtung der Austrageinheit gegenüber der Basiseinheit ausgerichtet.

Eine andere bevorzugte Bauform ist insbesondere zur topischen Applikation von Flüssigkeiten vorgesehen. Ein solcher Austragkopf kann eine lateral angeordnete Austragöffnung aufweisen. Eine Austragrichtung der Austragöffnung schließt mit der Verlagerungsrichtung der Austrageinheit gegenüber der Basiseinheit in diesem Falle einen Winkel > 0° ein, vorzugsweise einen Winkel zwischen 5° und 100°, insbesondere einen Winkel zwischen 30° und 60° oder zwischen 80° und 100°. Bei einem solchen Austragkopf erfolgt das Niederdrücken der Austrageinheit vorzugsweise über eine an einer Oberseite des Austragkopfes vorgesehene Betätigungsfläche. Bei einem solchen Austragkopf ist vorzugsweise vorgesehen, dass die Austragöffnung an einem seitlich hervorragenden Stutzen vorgesehen ist, der nach außen über die Außenkontur der Balgfeder hinausragt.

Der Austragkopf und insbesondere seine Austragöffnung können für unterschiedliche Applikationsformen ausgebildet sein. Insbesondere vorzugsweise ist der Austragkopf für die Abgabe eines Sprühstrahls ausgebildet. Er kann hierfür eine Wirbelkammer aufweisen, durch die die austretende Flüssigkeit vor Austritt mit einem Drall versehen wird, der beim Austritt zu einem konischen Sprühstrahl führt. Alternativ kann der Austragkopf auch eine Mehrzahl feiner Düsenöffnungen zu Erzeugung eines Sprühstrahls aufweisen, insbesondere eine Düsenplatte, in der diese Düsenöffnungen vorgesehen sind.

Auch die Gestaltung als Tropfenspender mit Tropfenbildungsfläche stromabwärts der Austragöffnung zur Abgabe diskreter Einzeltropfen sowie die Gestaltung als Spender zur Abgabe eines unzerstäubten Jets sind möglich.

Wie oben bereits erwähnt, sind der Austragkopf und der Gesamtspender vorzugsweise dahingehend ausgebildet, dass sie ein einfaches Recycling gestatten.

Der Austragkopf bzw. der Flüssigkeitsspender besteht vorzugsweise zu mindestens 90%, vorzugsweise zu mindestens 95%, insbesondere vorzugsweise 99% aus Polyolefin-Kunststoffen. Insbesondere kann der Spender überwiegend oder nahezu vollständig aus Polyethylen bestehen.

Der erfindungsgemäße Flüssigkeitsspender weist einen Austragkopf der beschriebenen Art sowie einen Flüssigkeitsspeicher auf, der lösbar oder fest, gegebenenfalls sogar einstückig, mit der Basiseinheit verbunden ist. Der Flüssigkeitsspeicher ist vorzugsweise aus HDPE gefertigt.

Der Flüssigkeitsspender wird vom Hersteller üblicherweise mit leerem Flüssigkeitsspeicher an ein Abfüllunternehmen geliefert, das ihn mit Flüssigkeit befüllt, insbesondere einer pharmazeutischen oder kosmetischen Flüssigkeit. Im Falle einer pharmazeutischen Flüssigkeit ist diese vorzugsweise zur nasalen Applikation, oralen Applikation oder topischen Applikation vorgesehen.

Vorzugsweise wird eine pharmazeutische Flüssigkeit in den Flüssigkeitsspender eingefüllt, wobei hierunter auch insbesondere salzhaltige wässrige Lösungen zur Applikation in die Atemwege eines Patienten verstanden werden. Auch wässrige Lösungen in Form einer Ringerlösung sind umfasst, ebenso wie gepufferte Lösungen oder wässrige Lösungen mit mindestens einem der Zusätze Kohlenhydrate, ätherische Öle, Menthol und Pflanzenextrakte sowie wässrige Lösungen enthaltend Vitamine, Spurenelemente, Mangan oder Zink, oder Zimtöl, Teebaumöl, Salbeiöl, Thymianöl, Zitronenmelissenöl.

Insbesondere kann die pharmazeutische Flüssigkeit auch eine nasal zu verabreichende Flüssigkeit mit abschwellender Wirkung sein, insbesondere eine Flüssigkeit mit einem Imidazolin-Bestandteil, wie beispielweise Oxymetazolin.

Neben dem Austragkopf und dem Flüssigkeitsspender als Ganzem betrifft die Erfindung auch ein Verfahren zur Herstellung zweier Arten von Austragköpfen. Das Verfahren dient dem Zweck, die beiden Arten von Austragköpfen besonders günstig herstellen zu können, indem viele Gleichteile verwendet werden.

Die Austragköpfe der ersten Art verfügen über eine außenliegende Balgfeder als Rückstellfeder. Vorzugsweise handelt es sich um Austragköpfe der oben beschriebenen Art.

Die Austragköpfe der zweiten Art weisen keine außenliegende Balgfeder aus Kunststoff auf, sondern eine innenliegende Schraubenfeder, insbesondere eine metallische Schraubenfeder. Bei der ersten Art ist eine solche Schraubenfeder nicht vorgesehen.

Die Austragköpfe beider Arten weisen eine baugleiche Basiseinheit zur Anbringung an einem Flüssigkeitsspeicher und ein baugleiches Applikatorgehäuse auf, welches als Teil einer Austrageinheit gegenüber der Basiseinheit verlagerbar ist und von einer Austragöffnung durchdrungen ist. Vorzugsweise unterscheiden sich die Austrageinheit und die Basiseinheit beider Arten von Austragköpfen außer durch die verwendete Feder zusätzlich maximal durch unterschiedliche Fingerauflagen, während alle anderen Bauteile baugleich sind.

Die Basiseinheit und das Applikatorgehäuse umgeben gemeinsamen einen Federaufnahmeraum, also einen Raum, in den die metallische Schraubenfeder mit insbesondere vorzugsweise zylindrischer Grundform im Falle des Austragkopfes der zweiten Art aufgenommen wird. Der Federaufnahmeraum wird vorzugsweise endseitig durch jeweils ringförmige Anlageflächen an der Basiseinheit und der Austrageinheit begrenzt, wobei die Anlageflächen vorzugsweise innenseitig und/oder außenseitig durch Führungsflächen flankiert sind, um die Schraubenfeder in Position zu halten.

Bei Austragköpfen der ersten Art ist dieser Federaufnahmeraum auch vorgesehen. Er verbleibt jedoch leer, da die Rückstellung durch die außenliegende Balgfeder verursacht wird. Nur bei Austragköpfen der zweiten Art wird hier eine metallische Schraubenfeder eingesetzt.

Bei den Austragköpfen der ersten Art wird stattdessen die außenliegende Kunststofffeder angebracht, vorzugsweise durch Anbringung einer Fingerauflage, an der die Balgfeder bereits zuvor befestigt wurde oder an der die Balgfeder einstückig angebracht ist.

### KURZBESCHREIBUNG DER ZEICHNUNGEN

Weitere Vorteile und Aspekte der Erfindung ergeben sich aus den Ansprüchen und aus der nachfolgenden Beschreibung von bevorzugten Ausführungsbeispielen der Erfindung, die nachfolgend anhand der Figuren erläutert sind.
Fig. 1 bis 5 und 6A zeigen ein erstes Ausführungsbeispiel eines erfindungsgemäßen Flüssigkeitsspenders bzw. von dessen Austragkopf.
Fig. 6B bis 6D zeigen bauliche Alternativen zur Gestaltung der Fig. 1 bis 6A.
Fig. 7 bis 9B zeigen ein zweites Ausführungsbeispiel eines erfindungsgemäßen Flüssigkeitsspenders bzw. von dessen Austragkopf.
Fig. 10 verdeutlicht, wie verschiedene Arten von Austragköpfen auf Basis weitgehend baugleicher Komponenten erzeugt werden.

### DETAILLIERTE BESCHREIBUNG DER AUSFÜHRUNGSBEISPIELE

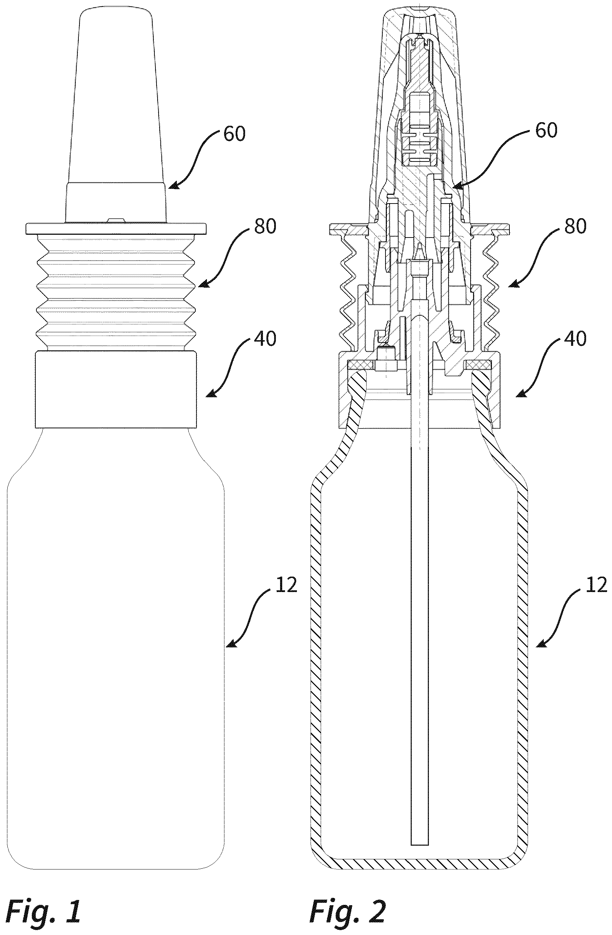

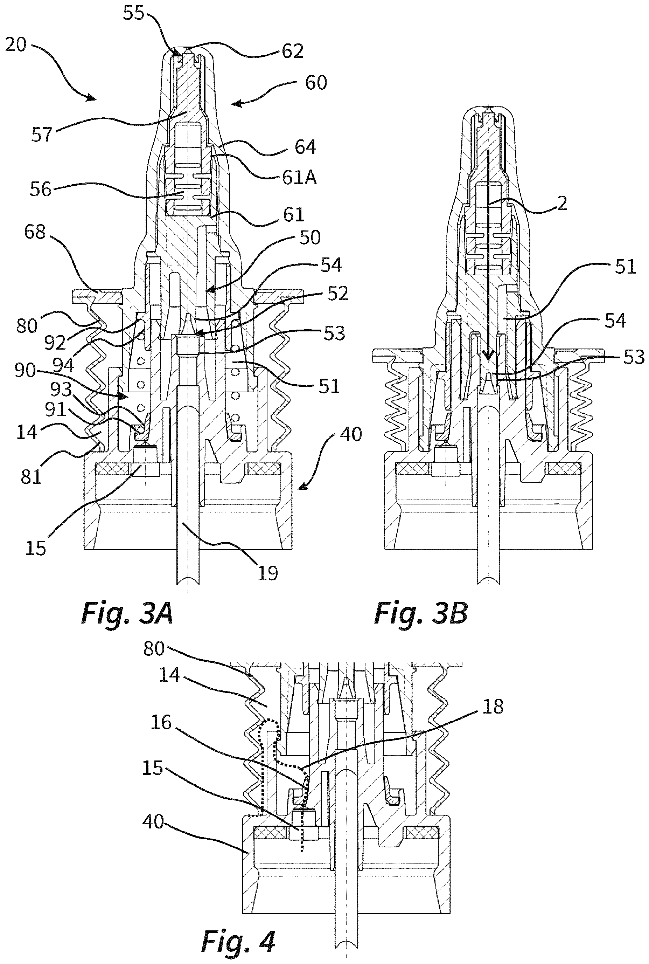

Die Fig. 1 und 2 zeigen das erste Ausführungsbeispiel eines erfindungsgemäßen Flüssigkeitsspenders 10 in geschnittener und ungeschnittener Ansicht. Die Fig. 3A bis 5 zeigen den Austragkopf 20 dieses Flüssigkeitsspenders 10, wobei in den Fig. 3A und 3B der unbetätigte und der betätigte Zustand dargestellt sind.

Der Flüssigkeitsspender 10 verfügt über einen Flüssigkeitsspeicher 12 in Form einer aus HDPE gefertigten Kunststoffflasche sowie über den am Flaschenhals des Flüssigkeitsspenders befestigten Austragkopf 20. Der Austragkopf 20 verfügt seinerseits über zwei gegeneinander entlang einer Pumprichtung 2 verlagerbare Hauptkomponenten, nämlich über eine Basiseinheit 40, die am Flaschenhals des Flüssigkeitsspeichers 12 angebracht ist, sowie über eine Austrageinheit 60, die entlang der Pumprichtung 2 gegenüber der Basiseinheit 40 verbunden ist.

Am distalen Ende der Austrageinheit 60 ist eine Austragöffnung 62 vorgesehen, durch die Flüssigkeit aus dem Flüssigkeitsspeicher 12 abgegeben werden kann.

Zur Förderung der Flüssigkeit vom Flüssigkeitsspeicher 12 bis zur Austragöffnung 62 weist der Austragkopf 20 eine Pumpeinrichtung 50 auf. Diese Pumpeinrichtung 50 verfügt über eine Pumpkammer 51, die über einen Einlasskanal mit einem Einlassventil 52 und mit einem Steigrohr 19 verbunden ist, welches in den Flüssigkeitsspeicher 12 ragt. Die Pumpkammer51 erstreckt sich bis zu einem Auslassventil 55, welches unmittelbar der Austragöffnung 62 vorgeschaltet ist.

Das Einlassventil 52 ist als Schieberventil ausgebildet. Es weist einen gestuften Ventilkanal 53 auf Seiten der Basiseinheit 40 sowie einen hierein einfahrenden Ventilschieber 54 auf Seiten der Austrageinheit 60 auf. Wird die Austrageinheit 60 niedergedrückt, wie es der Übergang von Fig. 3A zu Fig. 3B verdeutlicht, so fährt der Ventilschieber 54 in den Ventilkanal 53 ein und isoliert die Pumpkammer 51 gegenüber dem Flüssigkeitsspeicher 12, sobald der Ventilschieber 54 einen verjüngten Teil des Ventilkanals 53 erreicht. Ab diesem Zeitpunkt kann keine Flüssigkeit aus der Pumpkammer 51 mehr zurück in den Flüssigkeitsspeicher 12 gelangen, so dass das fortgesetzte Niederdrücken der Austrageinheit 60 einen Druckanstieg in der isolierten Pumpkammer 51 bewirkt.

Das Auslassventil 55 ist als Tip-Seal-Ventil ausgebildet. Dies bedeutet, dass es unmittelbar vor der Austragöffnung 62 schließt. Es verfügt über einen Ventilkörper 57 und eine Ventilfeder 56, wobei der Ventilkörper 57 und die Ventilfeder 56 als einstückiges Bauteil ausgebildet sind und mit dem Ende der Ventilfeder 56 in eine Aussparung eines Innenbauteils 61 der Austrageinheit 60 eingesetzt sind. Die Ventilfeder 56 wird durch einen Federrohrkörper gebildet, in dem horizontale Schlitze vorgesehen sind, um eine axiale Stauchung zu ermöglichen. Der Ventilkörper 57 ragt durch eine Wirbelkammer hindurch bis an die Austragöffnung 62 und verschließt diese in einem Ruhezustand.

Eine Druckerhöhung in der Pumpkammer 51 bewirkt eine Kraftbeaufschlagung des Ventilkörpers 57 nach unten, die geeignet ist, den Ventilkörper 57 zu verlagern, so dass das Auslassventil 55 geöffnet wird und Flüssigkeit durch die Austragöffnung 62 hindurch austreten kann. Durch die der Austragöffnung 62 vorgeschaltete Wirbelkammer tritt die Flüssigkeit in Form eines Sprühstrahls aus.

Das einstückige Bauteil, welches den Ventilkörper 57 und die Ventilfeder 56 bildet, ist an seiner Außenseite durch eine Gleitkante 61A des Innenbauteils 61 geführt. Diese Gleitkante 61A verursacht bei geschlossenem Auslassventil eine Haftreibung, durch die bewirkt wird, dass bei ansteigendem Druck das Auslassventil 55 nicht sofort öffnet, sondern erst leichtverzögert, dann jedoch schlagartig.

Hierdurch ist gewährleistet, dass beim Austrag unmittelbar der Ventilkörper 57 weit genug von der Austragöffnung 62 beabstandet ist, um einen bestimmungsgemäßen Austrag zu erzielen.

Das Niederdrücken der Austrageinheit 60 erfolgt über eine Fingerauflage 68, die beim Ausführungsbeispiel der Fig. 1 bis 5 außenseitig mittels einer Rastverbindung an dem Applikatorgehäuse 64 angebracht ist. Der Benutzer legt üblicherweise seinen Zeigefinger und seinen Mittelfinger auf diese Fingerauflage 68, um mit ihr die Austrageinheit 60 gegenüber der Basiseinheit 40 niederzudrücken.

Entfällt diese Betätigungskraft, so kehrt die Austrageinheit 60 in ihre in Fig. 3A dargestellte Ausgangsstellung zurück. Dies erfolgt unter der Wirkung einer Kunststoff-Rückstellfeder 80, die in Art eines Faltenbalgs ausgebildet ist. Diese Kunststoff-Rückstellfeder 80 ist außenseitig am Austragkopf 20 vorgesehen und umhüllt das obere Ende der Basiseinheit 40 und das untere Ende der Austrageinheit 60.

Die Gestaltung des Austragkopfs mit Kunststoff-Rückstellfeder 80 gestattet es, den gesamten Austragkopf 20 aus Kunststoff zu fertigen. Da sowohl die Kunststoff-Rückstellfeder 80 als auch die Ventilfeder 56 aus Kunststoff gefertigt sind, kann auf die Verwendung metallischer Schraubenfedern verzichtet werden. Vorzugsweise sind alle Bauteile oder bezogen auf die Masse zumindest 90% des Austragkopfes 20 aus Polyolefin-Kunststoffen hergestellt, insbesondere aus unterschiedlichen Sorten von Polyethylen.

Wie anhand der Fig. 3A zu erkennen ist, verfügt der Austragkopf 20 über einen Federaufnahmeraum 90, der zur Aufnahme einer metallischen Schraubenfeder vorgesehen ist, bei der Konfiguration des Spenders gemäß den Fig. 1 bis 5 jedoch frei ist von einer solchen metallischen Rückstellfeder. Allerdings gestattet es dieser Federaufnahmeraum 90, unter Verwendung vieler Gleichteile einen Austragkopf 20 ähnlich dem hier beschriebenen zur Verfügung zu stellen, der nicht über eine außenliegende Kunststoff-Rückstellfeder 80 verfügt, sondern stattdessen über eine innenliegende metallische Schraubenfeder. Dies wird im Weiteren auch noch gezeigt.

Wenngleich die Verwendung einer Kunststoff-Rückstellfeder 80 im Hinblick auf das Recycling erhebliche Vorteile bietet, ist hiermit üblicherweise nicht eine in gleichem Maße starke und relaxationsfreie Rückstellung durch eine metallische Schraubenfeder gewährleistet. Die Wahl der Gestaltung der Pumpeinrichtung50trägtdem Rechnung. Da der bereits beschriebene Ventilschieber 54 im Zuge der Betätigung zunächst einige Millimeter nach unten verlagert werden muss, bevor er das Einlassventil 52 schließt, kommt es bei einem Spender der dargestellten Art nicht in hohem Maße darauf an, dass die Kunststoff-Rückstellfeder 80 tatsächlich die Austrageinheit 60 bis in die oberste Endlage der Fig. 3A drückt. Auch wenn eine beispielsweise aufgrund langer Lagerzeit teilweise relaxierte Kunststoff-Rückstellfeder 80 Verwendungfindet, reicht diese üblicherweise aus, um den Ventilschieber 54 zusammen mit derAustrageinheit 60 so weit nach oben zu drücken, dass der Ventilschieber 54 aus dem verjüngten Teil des Ventilkanals 53 herausgezogen wird. Dies reicht für eine ordnungsgemäße Funktion der Pumpeinrichtung50 aus.

Die Fig. 3B verdeutlicht, dass beim Niederdrücken der Austrageinheit 60 der Ventilschieber 54 in seiner Endlage den verjüngten Teil des Ventilkanals 53 wieder verlässt, so dass in diesem Augenblick der Überdruck in der Pumpkammer 51 schlagartig abgebaut wird und der Austrag endet. Die Austragmenge der Pumpeinrichtung 50 ist alleine durch die Länge des verjüngten Teils des Ventilkanals 53 bestimmt.

Bei der Rückkehr des Austragkopfes 20 aus dem betätigten Zustand der Fig. 3B in den unbetätigten Zustand der Fig. 3A wird Flüssigkeit durch das Steigrohr 19 in die Pumpkammer 51 eingesogen. Hierdurch entsteht im Flüssigkeitsspeicher 12 ein Unterdruck, der durch einen Belüftungskanal ausgeglichen wird. Der entsprechende Belüftungspfad 18 ist in Fig. 4 dargestellt. Der Belüftungspfad verläuft von außen nach innen zunächst durch einen Spalt, den die als Balgfeder ausgestaltete Kunststoff-Rückstellfeder 80 mit dem Gehäuse der Basiseinheit 40 bildet. Der Belüftungspfad 18 verläuft weiter durch eine Führung der Austrageinheit 60 und der Basiseinheit 40 bis in einen Kapillarkanal 16, der durch den Basiskörper der Basiseinheit 40 und eine hierauf aufgeschobene Hülse gebildet ist, bis hin zu einer Filtereinheit 15, in der einströmende Luft von Bakterien befreit wird.

Von besonderer Bedeutung ist hierbei, dass der Belüftungspfad 18 durch eine Belüftungskammer 14 verläuft, die außenseitig durch die als Balgfeder ausgestaltete Kunststoff-Rückstellfeder 80 begrenzt ist. Wie eingangs bereits beschrieben, erfolgt das Einströmen der Luft durch einen Spalt am unteren Ende der Kunststoff-Rückstellfeder 80. Es handelt sich hierbei um einen sehr schmalen Spalt, so dass die durch die Flüssigkeit im Flüssigkeitsspeicher 12 befeuchtete Luft in der Belüftungskammer 14 nur in einem geringen Maße in die Umgebung austreten kann.

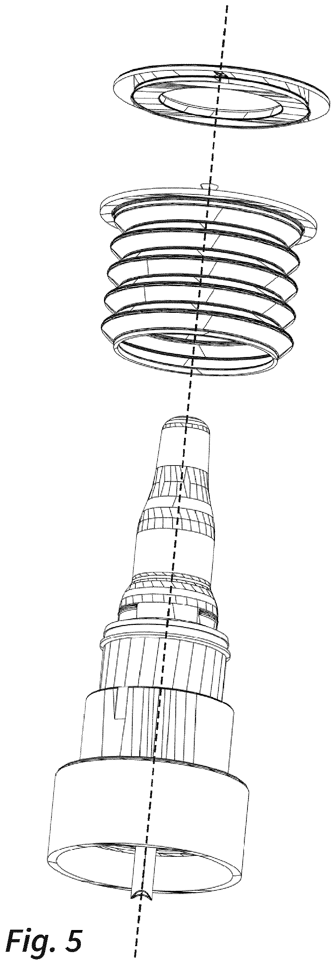

Fig. 5 zeigt nochmals den Austragkopf 20, wobei hier verdeutlicht ist, dass der Austragkopf 20 als Ganzes bereits montiert sein kann, wenn abschließend die Kunststoff-Rückstellfeder 80 sowie die Fingerauflage 68 montiert werden.

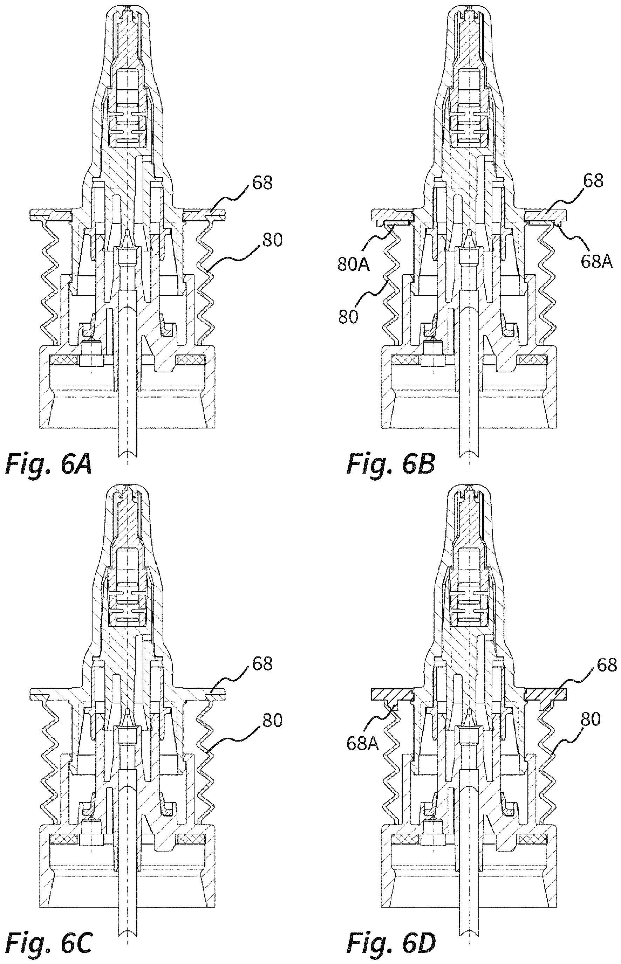

Die Fig. 6A bis 6D zeigen verschiedene Gestaltungen der Fingerauflage 68 und der Kunststoff-Rückstellfeder 80.

Fig. 6A zeigt die bereits in den vorangegangenen Figuren dargestellte Version. Hier sind die Fingerauflage 68 und die Kunststoff-Rückstellfeder 80 als einstückiges Bauteil realisiert, welches durch Zweikomponenten-Spritzguss hergestellt ist. Dies bedeutet, dass während der Montage die Balgfeder mittelbar über die Fingerauflage 68 gehandhabt und an der Basiseinheit 40 und der Austrageinheit 60 angebracht werden kann.

Bei der Gestaltung gemäß Fig. 6B ist vorgesehen, dass die Kunststoff-Rückstellfeder 80 und die Fingerauflage 68 getrennte Bauteile bilden. Die Kunststoff-Rückstellfeder 80 weist an ihrem oberen Ende einen nach innen weisenden umlaufenden Kragen 80A auf, der einen gegenüber den Falten des Balgs verringerten Innendurchmesser aufweist. Ein solcher Kragen 80A hilft bei der Entformung des Faltenbalgs während der Herstellung. Die Fingerauflage 68 ist im Falle der Darstellung der Fig. 6B mit einem umlaufenden Zentrierrand 68A versehen, der die Lagestabilität der Kunststoff-Rückstellfeder 80 gewährleistet. Da der Kragen 80A bis zum Applikatorgehäuse 64 nach innen weist und somit eigenständig eine Zentrierung bewirkt, ist eine solche zusätzliche Zentrierung jedoch nicht zwingend erforderlich.

Fig. 6C zeigt eine Gestaltung, bei der der Faltenbalg wie bei Fig. 6A einstückig mit der Fingerauflage 68 ausgebildet ist. Die Besonderheit ist hier, dass die Fingerauflage 68 einstückig mit dem Applikatorgehäuse 64 ausgebildet ist. Dies bedeutet, dass bei der Montage durch die Anbringung der Austrageinheit 60 an der Basiseinheit 40 auch die Fingerauflage 68 und die Kunststoff-Rückstellfeder 80 montiert werden.

Die Gestaltung der Fig. 6D ähnelt jener der Fig. 6B. Auch hier ist der Faltenbalg als separates Bauteil ausgebildet. Die Fingerauflage 68 weist auch hier einen Zentrierring 68A auf, der allerdings in diesem Fall von innen die Kunststoff-Rückstellfeder 80 zentriert.

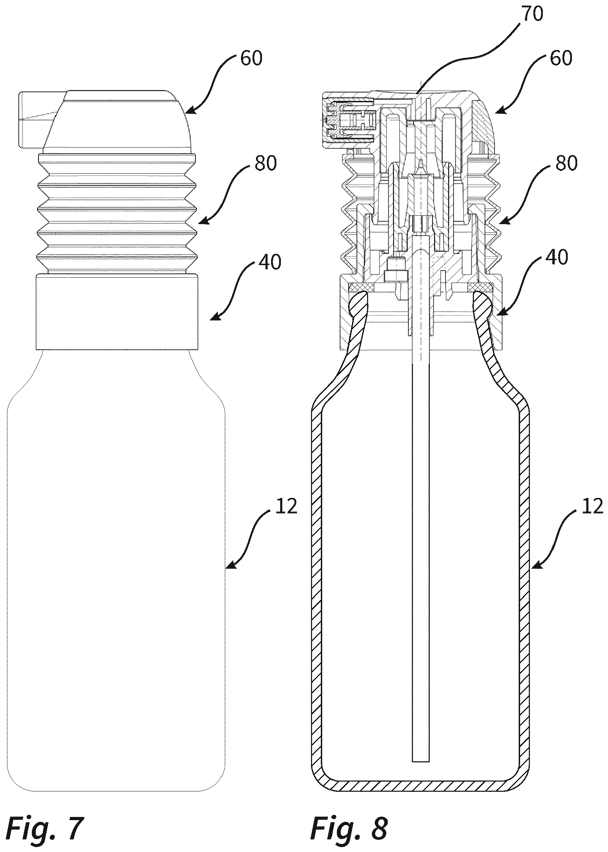

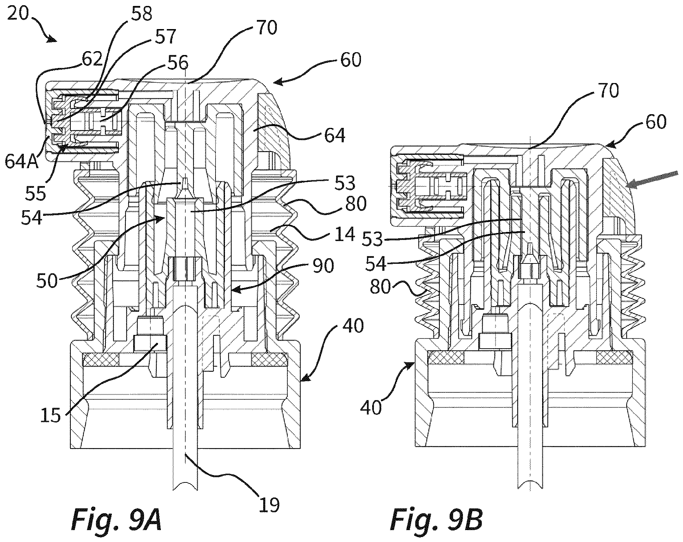

Die Fig. 7 bis 9B zeigen einen anders gearteten Flüssigkeitsspender 10, der sich durch die grundsätzliche Formgebung von der Gestaltung der Fig. 1 bis 6 unterscheidet. Die Fig. 7 und 8 zeigen den Flüssigkeitsspenders 10 in geschnittener und ungeschnittener Ansicht. Die Fig. 3A und 3B zeigen den Austragkopf 20 dieses Flüssigkeitsspenders 10 in unbetätigtem und betätigtem Zustand.

Der hier dargestellte Flüssigkeitsspender 10 ist zur topischen Anwendung vorgesehen. Er dient dem Zweck, Flüssigkeit auf der Haut eines Patienten aufzubringen.

Aus diesem Grund ist die Austragöffnung 62 bei dieser Gestaltung auch seitlich am Austragkopf 20 vorgesehen. Die Austragrichtung schließt mit einer Betätigungsrichtung 2, in der die Austrageinheit 60 gegenüber der Basiseinheit 40 verlagerbar ist, einen Winkel von 90° ein. Während die Pumpkammer und das Einlassventil weitgehend identisch zur Gestaltung der Fig. 1 bis 6 ausgebildet sind, ist das Auslassventil 55 bei dieser Gestaltung ebenso wie die Austragöffnung 62 seitlich vorgesehen. Zudem ist vorgesehen, dass die Abdichtung zwischen dem Ventilkörper 57 und der Austrageinheit 60 durch Dichtlippen 58 erfolgt, die am Ventilkörper 57 vorgesehen sind. Der Ventilkörper 57 und die Ventilfeder 56 sind in einem Aufnahmeraum angeordnet, der durch ein Verschlussteil 64A des Applikatorgehäuses 64 verschlossen ist.

Die Betätigung erfolgt mittels einer an der Oberseite der Austrageinheit 60 vorgesehenen Betätigungsfläche 70.

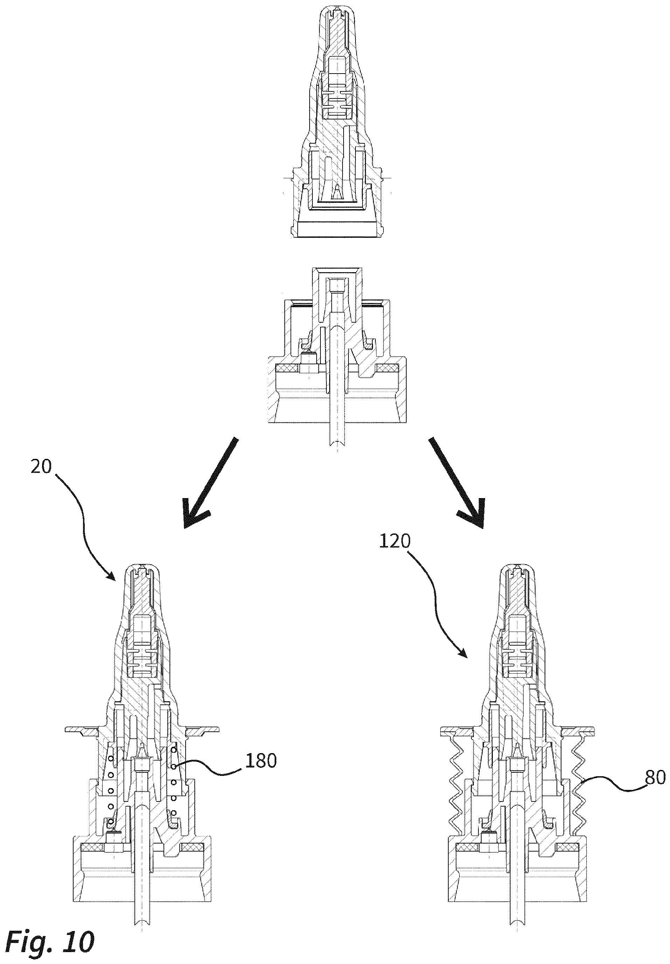

Anhand der Fig. 10 wird nochmals verdeutlicht, wie der dargestellte Austragkopf 20, insbesondere jener der Fig. 1 bis 5, Grundlage für eine Produktpalette mit unterschiedlichen Austragköpfen sein kann. Fig. 10 zeigt hierzu in der oberen Figurenhälfte die beiden maßgeblichen Teileinheiten des Austragkopfs 20, die Basiseinheit 40 sowie die Austrageinheit 60, wobei die Austrageinheit 60 noch nicht mit einer Fingerauflage 68 versehen ist.

Diese beiden Teileinheiten 40, 60 sind für zwei Gestaltungen des Austragkopfs 20 identisch, nämlich für die links in der Figur dargestellte Variante mit einer internen metallischen Schraubenfeder als Rückstellfeder 180 sowie für die in Fig. 10 rechtsseitige Variante, die jener der Fig. 1 bis 5 entspricht.

Diese Wiederverwendbarkeit der in Fig. 10, oben, dargestellten Baugruppen führt zu verringerten Kosten. Weiterhin erleichtert dies auch die Logistik, da die Baugruppen der Fig. 11, oben, vorgefertigt gelagert werden können und je nach Auftragslage für Austragköpfe mit metallischer Schraubenfeder und für vorteilhaft recycelbare Austragköpfe mit außenliegender Balgfeder verwendet werden können.

## Patentansprüche

1. Austragkopf (20) für pharmazeutische oder kosmetische Flüssigkeiten mit den folgenden Merkmalen:
a. der Austragkopf (20) weist eine Basiseinheit (40) zur Anbringung an einem Flüssigkeitsspeicher (12) auf, und
b. der Austragkopf (20) weist eine Austrageinheit (60) auf, die überein Applikatorgehäuse (64) verfügt, welches von einer Austragöffnung (62) durchdrungen ist, und
c. die Austrageinheit (60) ist gegen die Kraft einer Rückstellfeder (80) gegenüber der Basiseinheit (40) verlagerbar, wobei eine Pumpeinrichtung (50) des Austragkopfes (20) durch die Bewegung der Austrageinheit (60) gegenüber der Basiseinheit (40) betätigbar ist und in Reaktion auf die Betätigung Flüssigkeit aus dem Flüssigkeitsspeicher (12) zur Austragöffnung (62) fördert,
**gekennzeichnet durch** das folgende Merkmal:
d. die Rückstellfeder (80) ist als Kunststofffeder ausgebildet, vorzugsweise als Kunststoffbalgfeder.

2. Austragkopf (20) nach Anspruch 1 mit dem folgenden weiteren Merkmal:
a. die Rückstellfeder (80) ist an einer Außenseite der Austrageinheit (60) angebracht, vorzugsweise mit dem folgenden zusätzlichen Merkmal:
b. die Rückstellfeder umhüllt zumindest abschnittsweise Außenflächen der Basiseinheit (40) und der Austrageinheit (60).

3. Austragkopf (20) nach Anspruch 1 oder 2 mit den folgenden weiteren Merkmalen:
a. die Austrageinheit (60) verfügt über eine Fingerauflage (68) zur Kraftbeaufschlagung der Austrageinheit (60) in Richtung der Basiseinheit (40), und
b. die Rückstellfeder (80) wirkt auf Seiten der Austrageinheit (60) auf die Fingerauflage (68), insbesondere auf eine Unterseite der Fingerauflage (68),
vorzugsweise mit mindestens einem der folgenden Merkmale:
c. die Fingerauflage (68) weist eine ringförmige Form auf und umgibt das Applikatorgehäuse (64) außenseitig, und/oder
d. die Rückstellfeder (80) und die Fingerauflage (68) sind fest miteinander verbunden, so dass sie bei einer Montage des Spenders gemeinsam gehandhabt werden können, wobei die Rückstellfeder (80) und die Fingerauflage (68) vorzugsweise als einstückiges Bauteil ausgebildet sind, insbesondere als einstückiges Kunststoffbauteil aus mindestens zwei Materialien, die stoffschlüssig miteinander verbunden sind, oder
e. die Fingerauflage (68) und die Rückstellfeder (80) sind als getrennte Bauteile ausgebildet, wobei vorzugsweise an der Fingerauflage (68) und insbesondere vorzugsweise an einer Unterseite der Fingerauflage (68) eine Zentrierungsgeometrie (68A) vorgesehen ist, die eine Krafteinkopplungsfläche der Rückstellfeder (80) von innen und/oder außen führt, und/oder
f. die Fingerauflage (68) ist als vom Applikatorgehäuse (64) getrenntes Bauteil ausgebildet und an der Außenseite des Applikatorgehäuses (64) befestigt, insbesondere mittels einer Verrastung, oder
g. die Fingerauflage (68) in einstückig mit dem Applikatorgehäuse (64) ausgebildet.

4. Austragkopf (20) nach einem der vorstehenden Ansprüche mit den folgenden weiteren Merkmalen:
a. der Austragkopf (20) verfügt überein starres Bauteil (68), das fest an der Austrageinheit (60) oder der Basiseinheit (40) angebracht ist, und
b. die Rückstellfeder (80) ist einzig an diesem starren Bauteil (68) fest angebracht, insbesondere mittels einer einstückigen Verbindung.

5. Austragkopf (20) nach einem der vorstehenden Ansprüche mit den folgenden weiteren Merkmalen:
a. die Austrageinheit (60) ist über einen Gesamthubweg zwischen einer unbetätigten Ausgangslage und einer betätigten Endlage gegenüber der Basiseinheit (40) verlagerbar, und
b. die Pumpeinrichtung (50) ist derart ausgebildet und/oder derart an die Austrageinheit (60) gekoppelt, dass ausgehend von der Ausgangslage eine Verlagerung der Austrageinheit (60) in Richtung der betätigten Endlage für einen Teilhubweg keinen Flüssigkeitsaustrag bewirkt, wobei der Teilhubweg mindestens 3% des Gesamthubwegs beträgt.

6. Austragkopf (20) nach einem der vorstehenden Ansprüche mit den folgenden weiteren Merkmalen:
a. die Pumpeinrichtung (50) weist ein Einlassventil (52) auf, das als Schiebeventil ausgebildet ist, und
b. das als Schiebeventil ausgebildete Einlassventil (52) verfügt über einen Ventilkanal (53) und einen Ventilschieber (54), der zur umfänglichen Abdichtung mit einer Innenseite des Ventilkanals (53) ausgebildet ist.

7. Austragkopf (20) nach einem der vorstehenden Ansprüche mit den folgenden weiteren Merkmalen:
a. die Pumpeinrichtung (50) weist ein Auslassventil (55) auf, welches in Abhängigkeit eines Flüssigkeitsdrucks in einer Pumpkammer der Pumpeinrichtung (50) öffnet bzw. schließt, und
b. das Auslassventil (55) verfügt über einen Ventilkörper (57), der von einer Ventilfeder (56) in eine Schließposition gedrückt wird, und
c. die Ventilfeder (56) ist als Kunststofffeder ausgebildet,
vorzugsweise mit mindestens einem der folgenden zusätzlichen Merkmale:
d. das Auslassventil (55) verfügt über Ventilflächen, die bei geschlossenem Auslassventil (55) aneinander anliegen und zum Öffnen des Auslassventils (55) zueinander beabstandet werden, wobei die Ventilflächen am Ventilkörper (57) sowie am Applikatorgehäuse (64) vorgesehen sind, insbesondere unmittelbar an der zur Bildung der Austragöffnung (62) vorgesehenen Durchbrechung im Applikatorgehäuse (64), und/oder
e. die Ventilfeder ist als Balgfeder ausgebildet, oder
f. die Ventilfeder (56) wird durch einen Federrohrkörper gebildet, der mittels den Rohrkörper durchdringender Durchbrechungen eine elastische axiale Verkürzung gestattet, und/oder
g. das Auslassventil (55) ist derart ausgebildet, dass zu Beginn einer Öffnungsbewegung zum Zwecke des Öffnens ein Druckmaximum benötigt wird, wobei nach dem Öffnen das Auslassventil (55) mit gegenüber dem Druckmaximum verminderten Flüssigkeitsdruck offen gehalten werden kann.

8. Austragkopf (20) nach Anspruch 7 mit dem folgenden weiteren Merkmal:
a. die Ventilfeder (56) und der Ventilkörper (57) sind Teil eines einstückigen Bauteils.

9. Austragkopf (20) nach einem der vorstehenden Ansprüche mit den folgenden weiteren Merkmalen:
a. eine Austragrichtung der Austragöffnung (62) ist parallel zur Verlagerungsrichtung der Austrageinheit (60) gegenüber der Basiseinheit (40) ausgerichtet oder
b. eine Austragrichtung der Austragöffnung (62) schließt mit der Verlagerungsrichtung der Austrageinheit (60) gegenüber der Basiseinheit (40) einen Winkel > 0° ein, vorzugsweise einen Winkel zwischen 5° und 100°, insbesondere einen Winkel zwischen 30° und 60° oder zwischen 80° und 100°.

10. Austragkopf (20) nach einem der vorstehenden Ansprüche mit den folgenden weiteren Merkmalen:
a. der Austragkopf (20) weist einen Belüftungspfad (18) auf, entlang dessen Ausgleichsluft aus einer umgebenden Atmosphäre in den Flüssigkeitsspeicher (12) strömen kann, und
b. der Belüftungspfad (18) erstreckt sich durch eine Belüftungskammer (14), die außenseitig durch die Rückstellfeder (80) umgeben ist,
vorzugsweise mit mindestens einem der folgenden Merkmale:
c. im Belüftungspfad (18) ist eine Filtereinheit (15) vorgesehen, vorzugsweise bezogen auf eine Einströmrichtung der Luft stromabwärts der Belüftungskammer (14), und/oder
d. der Belüftungspfad ist zumindest abschnittsweise durch einen Kapillarkanal (16) gebildet, der stromaufwärts oder stromabwärts der Belüftungskammer (14) vorgesehen sein kann, und/oder
e. die Rückstellfeder (80) ist als Balgfeder ausgebildet, wobei die Balgfeder eine Belüftungsöffnung definiert, die einen lichten Querschnitt von mindestens 1 mm² aufweist, insbesondere vorzugsweise von mindestens 5 mm².

11. Austragkopf (20) nach einem der vorstehenden Ansprüche mit dem folgenden weiteren Merkmal:
a. die Rückstellfeder umgibt einen Luftfederraum (14), innerhalb dessen Luft enthalten ist, die bei Verlagerung der Austrageinheit (60) an die Basiseinheit (40) komprimiert wird.

12. Austragkopf (20) nach einem der vorstehenden Ansprüche mit den folgenden weiteren Merkmalen:
a. der Austragkopf (20) ist als Austragkopf (20) zur nasalen Applikation vorgesehen und das Applikatorgehäuse (64) weist eine längliche und sich zu einem distalen Ende hin verjüngende Formgebung auf, an deren distalem Ende die Austragöffnung (62) vorgesehen ist, wobei vorzugsweise eine das Applikatorgehäuse umgebende Fingerauflage (68) vorgesehen ist, oder
b. der Austragkopf (20) ist als Austragkopf (20) zur topischen Applikation vorgesehen und weist eine lateral angeordnete Austragöffnung (62) auf, wobei vorzugsweise an einer Oberseite des Austragkopfes eine Betätigungsfläche (70) vorgesehen ist.

13. Austragkopf (20) nach einem der vorstehenden Ansprüche mit mindestens einem derfolgenden weiteren Merkmale:
a. der Austragkopf (20) ist als Sprühkopf ausgebildet, und/oder
b. die Rückstellfeder (80) ist von einem Flüssigkeitspfad vom Flüssigkeitsspeicher (12) zur Austragöffnung (62) getrennt und ist nicht in Flüssigkeitskontakt, und/oder
c. der Austragkopf (20) besteht zu mindestens 90%, vorzugsweise zu mindestens 95%, insbesondere vorzugsweise 99% aus Polyolefin-Kunststoffen, und/oder
d. die Rückstellfeder (80) ist aus einem thermoplastischen Elastomer hergestellt, und/oder
e. die Rückstellfeder (80) ist als Kunststoffbalgfeder ausgebildet, wobei ein letztes Balgsegment (81) auf Seiten der Basiseinheit (40) als sich in distale Richtungverjüngendes Balgsegment ausgebildet ist.

14. Austragkopf (20) nach einem der vorstehenden Ansprüche mit den folgenden weiteren Merkmalen:
a. zwischen der Basiseinheit (40) und der Austrageinheit (60) ist ein Federaufnahmeraum (90) vorgesehen, der zur Aufnahme einer Schraubenfeder ausgebildet ist, wobei vorzugsweise keine Schraubenfeder im Federaufnahmeraum (90) angeordnet ist, und
b. an der Basiseinheit (40) und der Austrageinheit (60) sind jeweils ringförmige Anlageflächen (91, 92) für die Schraubenfeder vorgesehen, wobei die ringförmigen Anlageflächen vorzugsweise innenseitig und/oder außenseitig durch Führungsflächen (93,94) begrenzt sind.

15. Flüssigkeitsspender (10) mit den folgenden Merkmalen:
a. der Flüssigkeitsspender (10) weist einen Flüssigkeitsspeicher (12) auf, und
b. der Flüssigkeitsspender (10) weist einen Austragkopf (20) nach einem der vorstehenden Ansprüche auf.

16. Flüssigkeitsspender (10) nach Anspruch 14 mit den folgenden weiteren Merkmalen:
a. der Flüssigkeitsspeicher (12) ist mit einer pharmazeutischen Flüssigkeit befüllt, insbesondere zur nasalen oder topischen Applikation, und/oder
b. der Flüssigkeitsspeicher (12) weist ein Innenvolumen von weniger als 100 ml auf, insbesondere von weniger als 50 ml, und/oder

17. Flüssigkeitsspender (10) nach Anspruch 15 oder 16 mit dem folgenden weiteren Merkmal:
a. der Flüssigkeitsspender (10) besteht zu mindestens 90%, vorzugsweise zu mindestens 95%, insbesondere vorzugsweise 99% aus Polyolefin-Kunststoffen,
vorzugsweise mit mindestens einem der folgenden zusätzlichen Merkmale:
b. der Flüssigkeitsspender (10) besteht zu mindestens 90%, vorzugsweise zu mindestens 95%, insbesondere vorzugsweise 99% aus Kunststoff, aus Polyethylen, und/oder
c. der Flüssigkeitsspeicher (12) besteht aus Hart-Polyethylen (HDPE).

18. Verfahren zur Herstellungzweier Arten von Austragköpfen mit den folgenden Merkmalen:
a. es werden Austragköpfe (20, 120) einer ersten Art hergestellt, die über eine außenliegende Balgfeder als Rückstellfeder (80) verfügen, wobei diese Austragköpfe vorzugsweise nach einem der Ansprüche 1 bis 13 ausgebildet sind, und
b. es werden Austragköpfe einer zweiten Art hergestellt, die über eine innenliegende Schraubenfeder als Rückstellfeder (180) verfügen, und
c. die Austragköpfe der ersten und der zweiten Art weisen eine baugleiche Basiseinheit (40) zur Anbringung an einem Flüssigkeitsspeicher (12) und ein baugleichesApplikatorgehäuse (64) auf, welches gegenüber der Basiseinheit (40) verlagerbar ist und von einer Austragöffnung (62) durchdrungen ist, und
d. bei Austragköpfen der ersten Art wird die Rückstellfeder (80) außenseitig angebracht, so dass sie das Applikatorgehäuse (64) und die Basiseinheit (40) zumindest abschnittsweise umhüllt, und
e. bei Austragköpfen der zweiten Art wird die Rückstellfeder (180) in einen Federaufnahmeraum (90) eingebracht, der außenseitig durch die Basiseinheit (40) und das Applikatorgehäuse (64) umgeben wird und der endseitig durch innenliegende ringförmige Anlageflächen (91, 92) der Basiseinheit (40) und des Applikatorgehäuses (64) begrenzt wird.
